# EUROPEAN PATENT SPECIFICATION

(11) **EP 0 756 011 B1**
(45) Date of publication and mention of the grant of the patent: **26.03.2003**
(21) Application number: 96111801.5
(22) Date of filing: 23.07.1996
(51) Int. Cl.: C13J 1/06, C12N 1/16, C12P 7/08

(54) **Process for the treatment of molasses**
Verfahren zur Behandlung von Melasse
Procédé de traitement de mélasses

(30) Priority: 27.07.1995 IT GE950084
(43) Date of publication of application: 29.01.1997
(73) Proprietor: MINISTERO DELL' UNIVERSITA' E DELLA RICERCA SCIENTIFICA E TECNOLOGICA, 00144 Roma (IT)
(72) Inventor: Vallini, Giorgio, I-16146 Genova (IT)
(74) Representative: Porsia, Attilio, Dr.

(56) References cited:
- FR-A- 2 692 908
- GB-A- 2 152 057
- US-A- 4 359 430
- US-A- 5 077 201

## Description

The present invention relates to a process for the treatment and for the processing of the molasses obtained as by-products in the sugar manufacture.

At the end of the processes for the extraction of the sugar, particularly from the sugar beets, the so-called molasses are obtained as a by-product of the crystallization phase of the raw juices, which consists in a dense liquid (concentration in Brix degrees (Bx) corresponding to about 80) in which still a substantial amount of sugar is present (amount of sugar (Qz) expressed in % on the weight of the dry substance equal to 50-60%) but which may be more extracted by crystallization with the traditional methods and which as such could find commercial placing only at a price corresponding to about one half of that paid by the sugar industries to the beet-growers for the purchase of the raw material. The said molasses where thus commonly used for fermentative processes of various kinds, like alcoholic, citric and the like fermentation. In this manner a considerable amount of commercially "noble" product, the sugar, was utilized as raw material for subsequent processing proper for its commercial convenience.

Techniques have been developed for the chromatographic treatment of the molasses, particularly of the sugar beets molasses, which allows the recovery of an important percentage of the sugar contained in it. The said techniques allows the separation of the molasses in substantially three fractions, one of which is rich in sugar, another rich in betaine (trimethyl glycine) and a third fraction presenting a low tenor of sugar. The said last fraction is at a concentration and at a purity degree very low as the sugar is concerned, so that its concentration presents great difficulties due to the high viscosity of the sugar solutions of this kind. From the other side the concentration of the residual sugar makes problematic any commercial placing.

From US-A-4.359.430 a process for the recovery of betaine from beet molasses is known, according to which the molasses diluted to 25-50% solids, are introduced to the top of a chromatographic column containing a cation exchange resin, are eluted with water and a fraction of betaine is collected from the downstream side of the resin. The betaine fraction is evaporated under vacuum and the betaine is crystallized. Nothing is said in this prior art patent about the further treatment processed of the non-sugar waste fraction and of the sugar containing fraction recovered from the column as eluate.

From US-A-5.077.201 a process is known for producing a natural blue pigment (indigo) by submerged fermentation of a mutant Morel mushroom fungus.

None of the above prior art documents is concerned with the alcoholic and yeast fermentation, nor with the fermentation on a substrate containing the chromatographic fractions produced during the separation process of betaine from the molasses.

Scope of the present invention is therefore to furnish a treatment process of the molasses which allows to recover from one side a substantial percentage of sugar from a first fraction of molasses by chromatographic treatment of same, and from the other side of re-using the fraction more poor in sugar coming from this treatment as diluent for a second fraction of molasses thus obtaining a diluted must which may be used as raw material for transformation processes as the fermentative processes which bring to the obtainment of a commercial product, as for instance ethylic alcohol, or yeast, and of a residue which is almost free from sugar and of more easy to be discharged.

Object of the present invention is therefore a process for the treatment of molasses obtained as by-product in the manufacture of sugar, comprising the steps of:
- chromatographic separation from a first fraction of molasses of a plurality of fractions, one of which at least rich in sugar, and one at low sugar content;
- mixing said fraction at low sugar content with a second fraction of molasses in order to obtain a diluent must at a dilution which is apt for a subsequent treatment of fermentation and/or of culture;
- using said diluted must as fermentation and/or culture substrate, and
- separation and if necessary purification of the product obtained from said treatment and discharge of the residue.

In the case in which the diluted must be subjected to fermentation, same will be advantageously of the alcoholic type, but is may also be a fermentation directed to the production for example of citric acid or of gluconic acid or of a biomass like the yeast for the bread manufacture or for other alimentary uses.

Further advantages and characteristics of the process according to the present invention will become apparent from the following description of a preferred embodiment of the present invention made, by way of example, with reference to the annexed single sheet of drawings, in which:

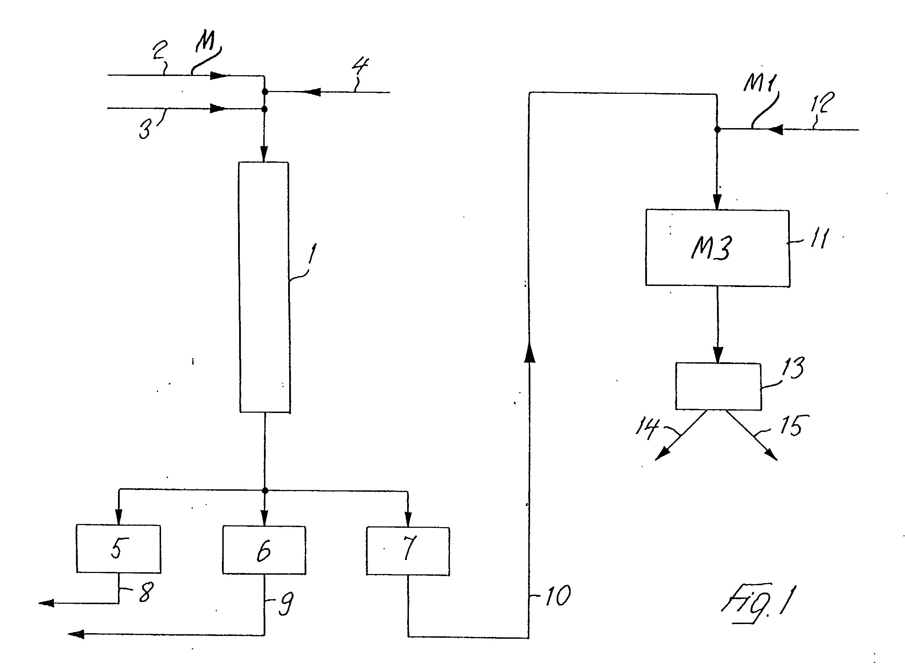

The figure 1 is a flow diagram which shows diagrammatically the plant for the carrying out of the process according to the invention.

With reference to the drawing, with numeral 1 a plant for the chromatographic separation is shown, comprising in a per se known manner, one or more chromatographic columns.

The molasses M, having common features obtained in the process of extraction of sugar from sugar beets, are fed at the head of this plant 1 through the conduit 2. By way of example, the said molasses M have a concentration expressed in Brix degrees of about 80, and contain from 55 to 60% of sugar with respect to the total dry substance.

The said molasses M are previously diluted with a comparatively small mount of water fed through the conduit 3 for a preliminary treatment, whilst from conduit 4 the elution water necessary to the chromatographic separation process is fed in the plant 1. The nature of the filling of the chromatographic separator 1, which is outside from the scope of the present invention and which by consequence will be not described here, permits the separation of the molasses in three fractions which will be respectively indicated as fraction I, II and III which will be collected respectively at the base of the plant 1 in the collectors 5, 6 and 7.

The fraction I is the one having the most elevated content in sugar, the fraction II contains the betaine, and the fraction III is the most diluted, which contains sugar in an amount notably minor with respect to the fraction I.

The fractions I and II are transferred through the conduits 8 and 9 to the further treatment (not shown nor described), whilst the fraction III is fed through the conduit 10 to the treatment vat 11, in which it is mixed with the molasses M1, similar to that M previously utilized in the first step of the process, which is fed through the conduit 12, thus obtaining a must M3 which is subjected to a further treatment which may be a fermentation treatment, or a feeding treatment for a yeast culture. The raw product originated in 11 is separated in the separator 13 in the desired products. In the instance in which the must was subjected to alcoholic fermentation the said separation process will be a distillation process, with obtainment of the azeotrope water-alcohol, which will be discharged through the conduit 14, and the distillation residual called distiller's wash, which will be discharged through the conduit 15.

Obviously, in the case that the must M3 be utilized as feeding liquid for a culture of saccharomycetes, the product discharged through 14 will be a biomass.

The amount of water added both as dilution and as elution to the molasses M through the conduits 3 and 4 is calculated so as to obtain the maximum result both from the point of view of the separation of the fractions I, II and III and from the point of view of their concentration. To this end it may be said that by varying the parameters it is possible to increase the concentration of the fraction I and thus to obtain the maximum yield of sugar, by however lowering the concentration of the fraction III. The fraction I generally contains about the 25% of sugar (weight/volume) whilst the fraction III contains about the 3-4% (w/v). Since said fraction is assigned to the mixing with the molasses M1 to form the fermentation must M3, it is advantageous that the concentration value of the sugar in said must M3 be not lesser than 10,5% (w/v) and not higher than 30,0% (w/v). This in order to avoid to have residuals of non fermented sugars, and at the same time to obtain a wine having an acceptable concentration in alcohol (8-10% w/v). In the case that the must M3 is the feed must in the production of yeast, its concentration in sugar may be also lower, in the order of 5-15%, so as to improve the yield in biomasses.

The process according to the invention will be further shown by the following practical example, which refers to a plant according to the invention combined with a process of alcoholic fermentation.

### Example

In the chromatographic separator 1 are continuously fed, through the conduit 2, 170 tons/day (117 m³) of molasses M having Bx=80 and Qz=58,4. The said molasses M are diluted through the conduit 3 with 57 m³ of water/day and eluted through the conduit 4 with 873 m³ of water/day. The three fractions 5, 6 and 7 recovered in 5, 6 and 7 following the elution where formed by:
fraction 5: 218 m³ of liquid, containing 55,9 tons of sugar (25,6% w/v);
fraction 6: 218 m³ of liquid containing 10,3 tons of betaine, and 1.1 t of sugar;
fraction 7: 611 m³ of liquid containing 21.2 t of sugar (3.5% w/v).

The fractions 5 and 6 are fed through the conduits 8 and 9 to the further processing whilst the fraction 7 is fed in the vat 11 through the conduit 10, and in said vat it is mixed with 204 t/day (141 m³) of molasses M1, similar to that M, fed through the conduit 12. The mix (752 m³, 15,5 % in sugar w/v) is subjected in the vat 11 to alcoholic fermentation. There are obtained 753 m³ of a wine with the 9.3% of alcohol v/v, from which are distilled 13.700 Edri (hectoliters of anhydrous alcohol) which are discharged through the conduit 14, thus obtaining 683 m³ of distillation residue, discharged through conduit 15.

The thus conceived process, allows from one side the recovery of a substantial amount of sugar from the molasses, and from the other side the useful use of eluted fractions which otherwise would be the object of costly concentration and/or purification processes.

## Claims

1. Process for the treatment of molasses obtained as by-products in the sugar manufacture, comprising the steps of:
- chromatographic separation from a first fraction (M)of molasses of a plurality of fractions (5,6,7), one of which (5) at least rich in sugar, and one (7) at low sugar content;
- mixing said fraction (7) at low sugar content with a second fraction of molasses (M1) in order to obtain a diluted must (M3) at a dilution which is apt for a following treatment of fermentation;
- fermentation of said diluted must (M3), and
- separation and if necessary purification of the product obtained from said treatment and discharge of the residue.

2. Process according to claim 1, in which the said fermentation is an alcoholic fermentation.

3. Process according to claim 2, in which the concentration of sugar in the diluted must (M3) is comprised between 10,5% and 20.0% (weight/volume).

4. Process for the treatment of molasses obtained as by-products in the sugar manufacture, comprising the steps of:
- chromatographic separation from a first fraction (M) of molasses of a plurality of fractions (5,6,7), one of which (5) at least rich in sugar, and one (7) at low sugar content;
- mixing said fraction (7) at low sugar content with a second fraction of molasses (M1)in order to obtain a diluted must (M3) at a dilution which is apt for a following use as a culture substrate;
- use of said diluted must (M3) as a culture substrate, and
- separation and if necessary purification of the product obtained from said treatment and discharge of the residue.

5. Process according to claim 4, in which the said must (M3) feeds the yeast production.

6. Process according to claim 5, in which the concentration in sugar in the must (M3)is comprised between 5% and 15% (weight/volume).

## Revendications

1. Procédé de traitement de mélasses obtenues en tant que sous-produits dans la production des sucres, comprenant les stades de :
- séparation par chromatographie d'une première fraction (M) de mélasses parmi une pluralité de fractions (5, 6, 7), l'une (5) d'entre elles au moins étant riche en sucre, et une (7) ayant une basse teneur en sucre ;
- mélange de la fraction (7) à basse teneur en sucre à une deuxième fraction de mélasses (M1) afin d'obtenir un mou (M3) dilué à une dilution qui est apte à subir un traitement suivant de fermentation ;
- fermentation du mou (M3) dilué, et
- séparation et si nécessaire purification du produit obtenu par ce traitement et évacuation du résidu.

2. Procédé suivant la revendication 1, dans lequel la fermentation est une fermentation alcoolique.

3. Procédé suivant la revendication 2, dans lequel la concentration du sucre dans le mou (M3) dilué est comprise entre 10,5% et 20,0 % (poids/volume).

4. Procédé de traitement de mélasses obtenues en tant que sous-produits dans la production du sucre comprenant les stades de :
- séparation par chromatographie d'une première fraction (M) de mélasses parmi une pluralité de fractions (5, 6, 7), l'une (5) d'entre elles au moins étant riche en sucre, et l'une (7) ayant une basse teneur en sucre ;
- mélange de la fraction (7) à basse teneur en sucre à une deuxième fraction de mélasses (M1) afin d'obtenir un mou (M3) dilué à une dilution qui est apte à subir une utilisation suivante en tant que substrat de culture ;
- utilisation du mou (M3) dilué comme substrat de culture, et
- séparation et si nécessaire purification du produit obtenu par ce traitement et évacuation du résidu.

5. Procédé suivant la revendication 4, dans lequel le mou (M3) est une charge pour la production de levure.

6. Procédé suivant la revendication 5, dans lequel la concentration en sucre du mou (M3) est comprise entre 5 % et 15 % (poids/volume).

## Patentansprüche

1. Verfahren zur Behandlung von Melasse, die als Nebenprodukt bei der Zuckerherstellung anfällt, mit folgenden Schritten:
- chromatografische Abscheidung mehrerer Fraktionen (5, 6, 7) von einer ersten Melassefraktion (M), wobei wenigstens eine dieser Fraktionen (5) reich an Zucker ist und eine (7) einen niedrigen Zuckergehalt hat;
- Mischen dieser Fraktion (7) mit niedrigem Zuckergehalt mit einer zweiten Melassefraktion (M1) zur Gewinnung eines verdünnten Mosts (M3), dessen Verdünnung für eine nachfolgende Fermentierungsbehandlung geeignet ist;
- Fermentierung dieses verdünnten Mosts (M3), und
- Abscheidung und, wenn nötig, Reinigung des durch diese Behandlung gewonnenen Produkts und Entfernung des Rückstands.

2. Verfahren nach Anspruch 1, bei dem die Fermentierung alkoholische Gärung ist.

3. Verfahren nach Anspruch 2, bei dem die Zuckerkonzentration in dem verdünnten Most (M3) zwischen 10,5% und 20,0% (Raumgewicht) beträgt.

4. Verfahren zur Behandlung von Melasse, die als Nebenprodukt bei der Zuckerherstellung anfällt, mit folgenden Schritten:
- chromatografische Abscheidung mehrerer Fraktionen (5, 6, 7) von einer ersten Melassefraktion (M), wobei wenigstens eine dieser Fraktionen (5) reich an Zucker ist und eine (7) einen niedrigen Zuckergehalt hat;
- Mischen dieser Fraktion (7) mit niedrigem Zuckergehalt mit einer zweiten Melassefraktion (M1) zur Gewinnung eines verdünnten Mosts (M3) mit einer Verdünnung, die für eine nachfolgende Verwendung als Kultursubstrat geeignet ist;
- Verwendung dieses verdünnten Mosts (M3) als Kultursubstrat, und
- Abscheidung und, wenn nötig, Reinigung des durch diese Behandlung gewonnenen Produkts und Entfernung des Rückstands.

5. Verfahren nach Anspruch 4, bei dem der Most (M3) als Nährmittel für die Hefeproduktion dient.

6. Verfahren nach Anspruch 5, bei dem die Zuckerkonzentration in dem Most (M3) zwischen 5% und 15% (Raumgewicht) beträgt.
